Europäisches Patentamt

European Patent Office

Office européen des brevets

(19)

(11) Numéro de publication: **0 203 633**
**B1**

(12) **FASCICULE DE BREVET EUROPEEN**

(45) Date de publication du fascicule du brevet: **18.04.90**

(51) Int. Cl.⁵: **A 61 M 25/00**

(21) Numéro de dépôt: **86200677.2**

(22) Date de dépôt: **22.04.86**

(54) Dispositif implantable d'injection chronique d'une substance, notamment thérapeutique.

(30) Priorité: **21.05.85 FR 8508067**

(43) Date de publication de la demande:
**03.12.86 Bulletin 86/49**

(45) Mention de la délivrance du brevet:
**18.04.90 Bulletin 90/16**

(84) Etats contractants désignés:
**DE FR GB IT NL SE**

(56) Documents cités:
**EP-A-0 119 596**
**EP-A-0 141 625**
**US-A-2 688 964**

**Larousse Encyclopédique, volume 1, page 707**

(73) Titulaire: **APPLIED PRECISION LIMITED**
**100 Fetter Lane**
**London EC4A 1DD (GB)**

(72) Inventeur: **Lazorthes, Guy**
**26 rue d'Auriol**
**F-31400 Toulouse (FR)**

(74) Mandataire: **Barre, Philippe**
**Cabinet Barre-Gatti-Laforgue 95 rue des**
**Amidonniers**
**F-31069 Toulouse Cédex (FR)**

**Description**

L'invention concerne un dispositif destiné à être totalement implanté dans une zone sous-cutanée accessible du corps d'un patient en vue de constituer un site d'injection chronique d'une substance liquide (essentiellement thérapeutique).

La technique récente d'implantation de sites d'injection consiste à diaposer, au cours d'une intervention sous anesthésie locale, une chambre d'injection sous le tissu sous-cutané de façon que celle-ci soit accessible à travers la peau; cette chambre est reliée à un cathéter qui délivre la substance directement dans la région du corps concernée. Ces sites d'injection peuvent demeurer dans le corps du patient pendant des durées prolongées et permettent de supprimer des injections répétées intraveineuses, intra-artérielles, intrarachidiennes, intra-ventriculaires cérébrales ou intrapéritonéales et de les remplacer par de simples injections sous-cutanées.

Un premier type de dispositif actuellement sur le marché vise à injecter, par unité, une dose de substance thérapeutique (désignée ci-après par "embole"). Ces dispositifs comprennent un disque rigide notamment en acier inoxydable qui est surmonté par une membrane élastique en silicone formant un dôme déformable; la chambre d'injection est constituée par le volume intérieur de ce dôme et est reliée à un cathéter. Lors de l'implantation, le dôme est orienté vers la ligne cutanée pour être accessible, d'une part, au moyen d'une aiguille d'injection, d'autre part, manuellement à travers la peau.

Après implantation, la mise en oeuvre de ce type de dispositifs consiste à traverser la membrane au moyen d'une aiguille montée sur une seringue afin de déverser la substance thérapeutique à l'intérieur du dôme déformable formant la chambre (le disque métallique qui obture celle-ci servant de butée à l'extrémité de l'aiguille), puis, après retrait de l'aiguille, à presser manuellement le dôme élastique à travers la peau afin d'expulser la dose de substance vers le cathéter.

Toutefois, ce type de dispositifs présente plusieurs défauts. En premier lieu, ils permettent seulement d'assurer des injections d'emboles par unité et ne sont pas appropriés pour effectuer des perfusions de longue durée. En effet, le dôme élastique du dispositif est inapte à assurer un maintien stable d'une aiguille de perfusion. En outre, ces dispositifs ont une durée de vie relativement courte et, au terme de quelques centaines d'injection, leur dôme élastique est le siège de micro-infiltrations: le dispositif doit alors être changé, ce qui oblige à une nouvelle intervention chirurgicale. Au surplus, et c'est là un défaut majeur de ce type de dispositifs, on a pu constater que l'embole contenue dans la chambre d'injection formée par le dôme déformable n'est jamais totalement refoulé dans le cathéter lorsque l'opérateur exerce une pression manuelle sur le dôme, et ce, quelles que soient les précautions prises; la petite quantité variable de substance qui reste dans le dôme après pression rend imprécise la dose réellement injectée, ce qui peut être très préjudiciable dans certains traitements, en particulier lors de l'administration de principes actifs tels que opioïdes, neuro-médiateurs, antimitotiques qui nécessitent une prescription précise.

Un autre type de dispositif existant vise à permettre l'exécution de perfusion, mais ne possède pas de membranes actionnables manuellement pour l'injection d'emboles. Ces dispositifs sont constitués par une chambre cylindrique en acier inoxydable qui est fermée d'un côté par un fond en acier inoxydable et, de l'autre, par un septum permettant le passage étanche de l'aiguille de perfusion. Le défaut essentiel de ces dispositifs est que leur usage est limité à des perfusions, ces dispositifs ne permettant pas de distribuer, par unité, des emboles correspondant à une quantité précise prédéterminée de substance thérapeutique; or un tel besoin se fait ressentir dans de très nombreux traitements thérapeutiques (chimiothérapies au long cours, traitements de la douleur par injections de morphine épidurale ou intrathécale,...). De plus, la configuration de ces dispositifs oblige à une ponction perpendiculaire qui conduit à des difficultés pratiques pour maintenir l'aiguille en place pendant la perfusion et pour la relier au système d'alimentation externe; c'est en particulier le cas du dispositif décrit dans la demande EP-A-0 119 596. Par ailleurs, un autre défaut de ces dispositifs provient de leur coût relativement élevé et de leur encombrement nettement plus important que les dispositifs à dôme souple (notamment dans le sens de la hauteur), qui rend leur implantation moins facile et forme sous la peau une surépaisseur pouvant entraîner une gêne pour le patient.

La présente invention se propose de fournir un dispositif perfectionné qui puisse être utilisé de façon universelle aussi bien pour pratiquer des injections d'emboles que pour effectuer des perfusions prolongées.

Un autre objectif essentiel de l'invention est de fournir un dispositif apte à distribuer, lors d'une injection, une dose précise prédéterminée de la substance thérapeutique.

Un autre objectif de l'invention est de fournir un dispositif de durée de vie très accrue par rapport aux dispositifs à dôme élastique.

Un autre objectif est de fournir un dispositif peu onéreux et de faible volume, propre à être très facilement implanté en zone sous-cutanée.

Un autre objectif est de fournir un dispositif ne conduisant à aucun risque de perturbations ou d'artefacts lors d'explorations réalisées par des techniques d'imagerie moderne (tomodensitométrie, résonnance magnétique...).

A cet effet, le dispositif visé par l'invention, destiné à être implanté dans une zone sous-cutanée accessible en vue de constituer un site de perfusion et/ou d'injection chronique d'une substance liquide, est du type comprenant:

un corps rigide monobloc d'épaisseur plus faible que ses deux autres dimensions, et de forme permettant de le glisser dans la zone sous-cutanée,

2

ledit corps étant creusé dans son épaisseur d'une dépression concave arrondie, apte à faire office de réservoi de substance liquide,

le corps étant percé d'un canal débouchant dans le fond de la dépression dans la partie la plus profonde de celle-ci par un orifice de communication,

ledit canal s'étendant dans l'épaisseur du corps de façon à déboucher à l'extérieur sur le pourtour dudit corps,

un cathéter souple étant fixé de façon étanche par son extrémité dans ledit canal de façon à se prolonger à l'extérieur du corps,

le corps étant pourvu autour de la dépression d'une bordure périphérique,

une membrane élastique de type auto-étanche sensiblement plane à l'état de repos étant fixée de façon étanche sur ladite bordure périphérique,

la dépression s'ouvrant sur une face du corps et présentsnt une profondeur faible par rapport aux dimensions de sa section d'ouverture, de façon que la membrane vienne épouser le fond de la dépression lorsqu'elle est repoussée par l'index de l'utilisateur.

Ledit dispositif se caractérise en ce que la membrane est positionnée sur le corps en position inclinée par rapport à la face inférieure de celui-ci, et en ce que ledit corps présente une épaisseur décroissante en direction de l'orifice de communication du canal dans la dépression.

La dépression sus-évoquée qui définit la capacité de la chambre d'injection possède de préférence pour les modèles de base les plus courants, un volume compris entre 0,2 et 1,7 cm³, de façon que la capacité de la chambre corresponde, lorsque la membrane est au repos, à une dose unitaire de substance thérapeutique (dont la quantité est généralement comprise dans cette plage de valeurs).

Les caractéristiques de forme de cette dépression, dont deux exemples seront illustrés en détail plus loin, sont de préférence définies de sorte qu'elle corresponde à l'empreinte d'un index, de façon que la membrane vienne naturellement épouser le fond de la dépression lorsqu'elle est repoussée par l'index de l'opérateur.

Le corps du dispositif peut être moulé d'un seul tenant en matière synthétique riqide biocompatible, notamment polycarbonate ou silicone renforcé. Ce corps remplit essentiellement trois fonctions:

en premier lieu, il définit une forme de chambre d'injection permettant un refoulement complet de la substance qu'elle contient, grâce à la faculté d'appliquer, par pression, la membrane contre la totalité de la surface de la dépression dudit corps,

de plus, il sert de soutien, par se bordure périphérique, au pourtour de la membrane plane, lequel est fixé notamment par collage aur cette bordure,

enfin, il sert de butée à l'extrémité de l'aiguille d'injection ou de perfusion lors du remplissage de la chambre d'injection.

La membrane élastique est découpée à la forme de la bordure périphérique du corps dans une matière synthétique élastique biocompatible du type auto-étanche ("Self-Sealing"), notamment silicone. La forme plane de cette membrane améliore l'étanchéité de celle-ci après un grand nombre d'injections comme cela sera expliqué plus loin. De plus, cette forme plane combinée à la disposition de la membrane sur le corps et à la forme de la dépression de ce dernier conduit:

à une aptitude de la membrane à se déformer naturellement pour épouser le fond de la dépression du corps sous l'action d'une pression manuelle,

à une amélioration de la stabilité d'une aiguille de perfusion, permettant de pratiquer sans risque ce type d'injection.

Le dispositif de l'invention combine ainsi les qualités suivantes: utilisation universelle aussi bien pour des injections d'emboles que pour des perfusions, précision de la dose injectée, durée de vie prolongée due à une meilleure étanchéité après de multiples perforations, faible coût en raison de sa simplicité structurelle et de la faculté de mouler le corps, forme peu épaisse du dispositif facilitant son implantation.

Selon une autre caractéristique de l'invention, le corps et la membrane élastique sont entièrement recouverts d'un revêtement souple en une matière biocompatible, en particulier en silicone. Ce revêtement accroît encore l'étanchéité de la membrane après un grand nombre de perforations et améliore la biocompatibilité du dispositif.

D'autres caractéristiques, buts et avantages de l'invention ressortiront de la description qui suit en référence aux dessins annexés qui en présentent deux modes de réalisation préférentiels; sur ces dessins qui font partie intégrante de la description:

la figure 1 est une coupe d'un premier mode de réalisation par un plan longitudinal de symétrie AA',

la figure 2 en est une vue de dessus,

les figures 3, 4, 5 et 6 en sont des coupes transversales respectivement par des plans BB', CC', DD' et EE',

les figures 7a, 7b et 7c sont des schémas illustrant la mise en oeuvre du dispositif,

les figures 8 et 9 sont des vues d'un autre mode de réalisation, respectivement en coupe par un plan de symétrie BB' et en vue de dessus suivant la flèche V.

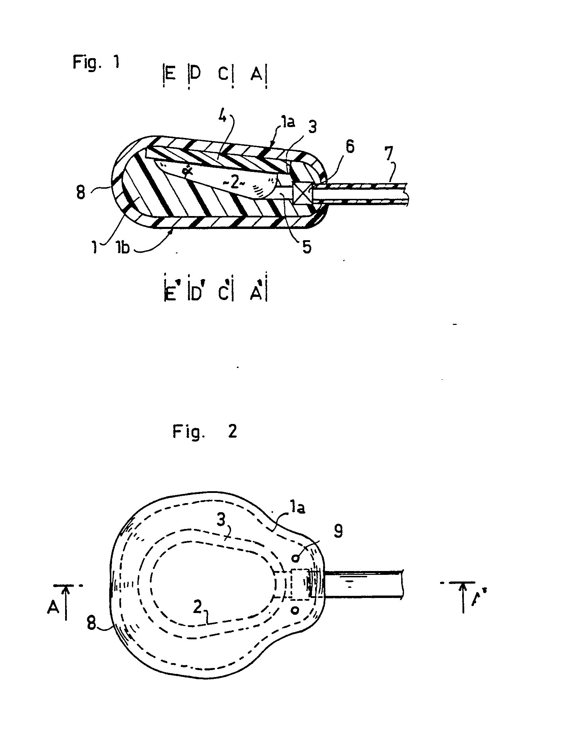

Le dispositif représenté à échelle 2 aux figures 1 à 7 est destiné à constituer un site d'injection chronique d'une substance dans une région déterminée du corps d'un patient.

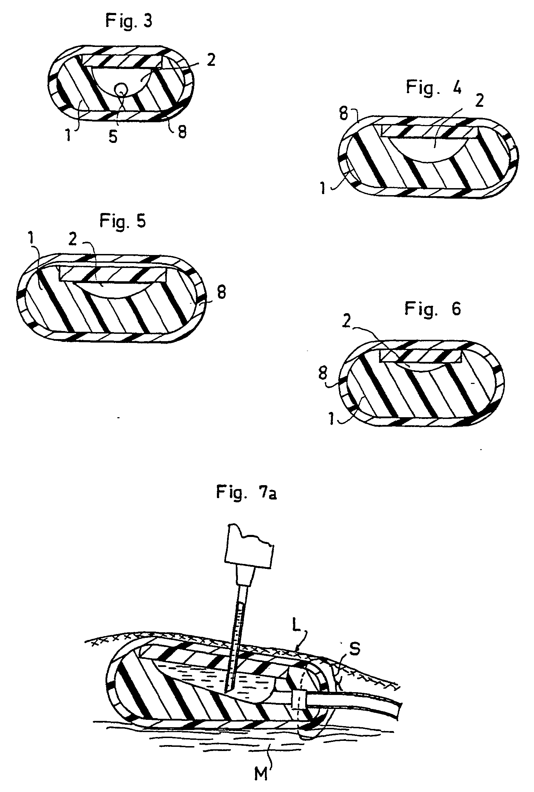

Ce dispositif comprend un corps rigide 1 moulé d'un seul tenant, en polycarbonate ou en silicone renforcé de "Dacron" ou polyéthérisulfome; ce corps présente une épaisseur de l'ordre de 10 mm, plus faible que ses autres dimensions. En vue de dessus, il possède une forme oblongue rétrécie sur un côté (côté droit à la figure 2): son chant périphérique est arrondi sur tout son pourtour.

La face supérieure la du corps, dite face active, est légèrement inclinée par rapport à la face opposée 1b, de sorte que le corps présente une épaisseur décroissante vers le côté rétréci.

Sur sa face active la, le corps 1 est creusé d'une dépression 2, de profondeur faible par rapport aux dimensions de sa section d'ouverture sur la face active.

Cette dépression possède une profondeur progressivement croissante vers sa portion la plus profonde située du côté rétréci du corps; elle présente une forme correspondant à l'empreinte d'un index: forme oblongue, symétrique par rapport au plan longitudinal AA' et se rétrécissant légèrement du côté rétréci du corps. De plus, comme le montrent les figures, la section de la dépression 2 par des plans de coupe parallèles à la face active possède une surface décroissante au fur et à mesure que ces plans de coupe se rapprochent du fond de la dépression.

En l'exemple, la portion centrale du fond de la dépression est de surface conique et se prolonge de chaque côté par des surfaces arrondies jusqu' à la section d'ouverture sur la face active; ce fond est incliné dans le sens longitudinal vers la partie la plus profonde, de façon à former, dans le plan de symétrie AA', un angle α avec la face active la sensiblement compris entre 10° et 15°.

Autour de la dépression 2, le corps est pourvu d'une bordure périphérique plane 3 sur laquelle est collé de façon étanche le pourtour d'une membrane élastique en silicone 4 du type auto-étanche qui ferme la dépression; cette membrane peut le cas échéant être du type "Silicone-Plug".

Cette membrane 4 est plane au repos et est inclinée comme la face active du corps; elle est découpée pour épouser la forme de la bordure périphérique 3; elle peut se déformer pour venir épouser le fond de la dépression sur toute sa surface.

La dépression 2 présente en l'exemple un volume de l'ordre de 1,3 à 1,7 cm$^3$ (par exemple 1,5 cm$^3$) et délimite ainsi une chambre d'injection dont la capacité maximale est égale à ce volume et peut être réduite jusqu'à une valeur sensiblement nulle par pression sur la membrane 4.

En outre, le corps 1 est percé, dans son épaisseur, d'un canal 5 qui s'étend dans le plan de symétrie entre la face active la et 1a face opposée 1b, à peu près parallèlement à cette dernière face 1b. D'un côté, ce canal débouche dans la dépression 2 au niveau de la partie la plus profonde de cette dernière, de façon à affleurer le fond de celle-ci; de l'autre côté, ce canal possède un tronçon de diamètre plus important qui débouche à l'extérieur du corps sur le pourtour de celui-ci.

Un filtre antibactérien 6 de type connu en soi (notamment filtre microporeux) est disposé dans le tronçon le plus large du canal 5, en appui contre l'épaulement que définit le changement de diamètre.

En outre, l'extrémité d'un cathéter souple 7 est fixée par collage dans ledit canal jusqu'au voisinage du filtre 6 (lequel est situé immédiatement en amont de cette extrémité). A la sortie du corps, ce cathéter 7 débouche selon une direction longitudinale approximativement parallèle au plan de 1a face 1b.

Par ailleurs, le corps 1 et la membrane 4 sont entièrement recouverts d'un revêtement souple en silicone 8. Ce revêtement est réalisé après collage de la membrane et du cathéter, par tout procédé connu: projection, trempage, moulage...

De part et d'autre de l'extrémité du cathéter 7, le corps et le revêtement sont percés de deux trous de suture tels que 9 qui traversent l'épaisseur du dispositif.

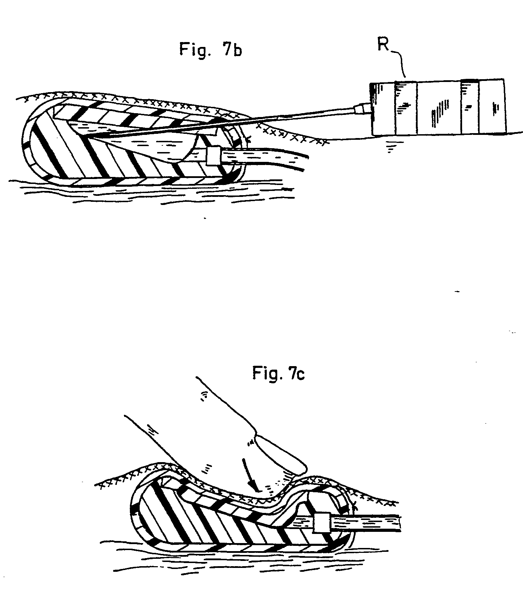

Le dispositif décrit est totalement implantable dans une zone sous-cutanée comme l'illustrent les figures 7a, 7b et 7c. Sa forme permet de le glisser facilement entre la ligne cutanée L et les tissus internes musculoaponévrotiques M dans une poche sous-cutanée pratiquée au cours de l'implantation. Deux sutures S traversant les trous 9 du dispositif immobilisent celui-ci et écartent les risques de torsion du cathéter 7 à sa sortie du corps.

La chambre d'injection délimitée par la dépression 2 et la membrane 4 au repos peut être remplie de liquide par injection à travers la peau, la membrane et son revêtement. Cette injection peut être réalisée de façon à disposer une dose de substance égale à la capacité de ladite chambre; il est également possible de piquer de façon stable une aiguille de perfusion afin de réaliser une perfusion prolongée d'un liquide sous une pression appropriée. Il est à noter qu'il est possible de piquer l'aiguille tangentiellement comme schématisé à la figure 7b; cet avantage important dans le cas d'une perfusion ne se retrouve ni dans les dispositifs à dôme (en raison des risques de traversée du dôme de part en part), ni dans les dispositifs à chambre cylindrique (en raison de la forme en hauteur de la chambre). Cet avantage est essentiel dans les perfusions nécessitant un maintien prolongé de l'aiguille de manière stable, tout en laissant le patient libre de ses mouvements: la disposition tangentielle de l'aiguille permet à l'ensemble du système externe S (seringue, pompe...) d'être appliqué au plan cutané et facilement fixé.

Le dispositif garde une étanchéité parfaite au terme d'un très grand nombre de perforations (quelques milliers); cette étanchéité est améliorée par rapport au dispositif à dôme souple pour deux raisons essentielles. En premier lieu, la matière élastique d'un dôme ayant subi de nombreuses perforations rapprochées a tendance à se déformer dans le sens centrifuge en bordure des trous, ce qui provoque des micro-infiltrations dans les zones où les trous sont très rapprochés; dans le dispositif de l'invention, la membrane 4, plane au repos, n'est pas affectée par ce phénomène. En outre, la double paroi formée par la membrane 4 et par le revêtement souple 8 crée au niveau des perforations des chevauchements de matière qui, à épaisseur totale égale, améliorent considérablement l'étanchéité.

Dans le cas de l'injection d'une embole, l'opérateur presse sur la membrane au moyen de son index jusqu'à ressentir le fond rigide du corps, comme l'illustre la figure 7c; la totalité de la dose est alors expulsée dans le cathéter, ce qui permet de pratiquer des injections de grande précision.

La simplicité structurelle du dispositif et la faculté de fabriquer le corps 1 par moulage lui confèrent un prix de revient abaissé par rapport aux dispositifs connus. En outre, sa biocompatibilité est excellente en raison de la nature des matériaux utilisés et de la présence du revêtement externe 8 (les fonctions de ce revêtement étant, d'une part, d'améliorer cette biocompatibilité, d'autre part, d'accroître l'étanchéité au terme d'un grand nombre de perforations). Le dispositif ne comporte aucun élément métallique et le patient peut faire l'objet d'explorations par des techniques d'imagerie moderne, sans risque de perturbations ou d'artefacts.

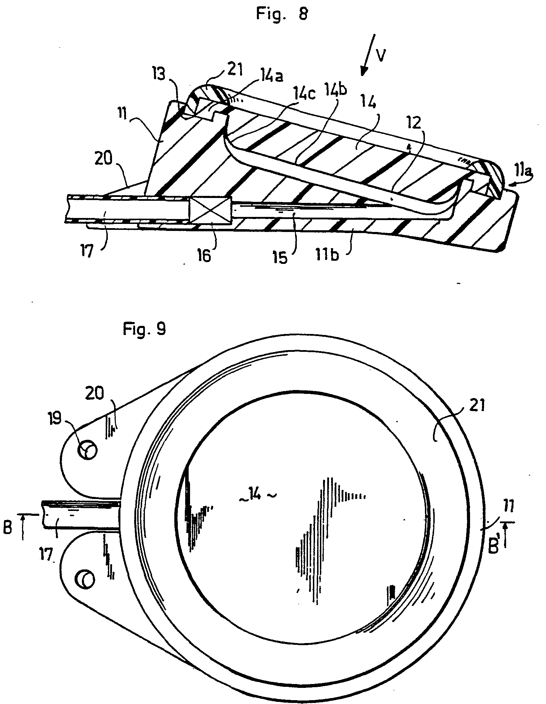

Les figures 8 et 9 représentent à échelle 3 une variante, dont les différences par rapport au dispositif précédent sont décrites ci-après.

Celle-ci possède un corps rigide 11 moulé d'un seul tenant de façon analogue au corps 1. Toutefois, en vue de dessus, ce corps présente une forme circulaire avec sur le côté le plus épais, deux petites extensions telles que 20 dotées de trous de suture 19.

Comme précédemment, la face supérieure 11a du corps est inclinée par rapport à la face opposée 11b, de sorte que le corps présente une épaisseur décroissante. (Du côté le moins épais, la face 11b présente une partie biaise afin d'avoir une épaisseur de matière suffisante).

Le corps 11 est creusé d'une dépression 12 dont le fond est parallèle à la face supérieure dans sa portion centrale et se raccorde à cette face par une portion périphérique concave arrondie.

Autour de la dépression 12, le corps est pourvu d'une bordure périphérique 13 qui est dotée d'une rainure sur tout son pourtour.

Une membrane élastique en silicone 14 ayant une bordure préformée 14a est collée sur le pourtour 13 avec un anneau de garde 21 collé au-dessus de l'ensemble.

La membrane 14 présente une épaisseur supérieure à la précédente. Au repos, sa face supérieure est plane, cependant que sa face inférieure est plane dans sa plus grande surface 14b et se raccorde au pourtour préformé 14a par une zone convexe arrondie 14c. La membrane 14 se déforme pour épouser le fond de la dépression 12 lorsqu'elle est repoussée par le doigt d'un opérateur.

En l'exemple, la dépression 12 présente un volume de l'ordre de 0,2 à 0,8 cm$^3$.

En outre, le corps est percé dans son épaisseur d'un canal 15 qui passe au-dessous de la dépression 12 et débouche au fond de celle-ci du côté du corps le moins épais.

Comme précédemment, le canal 15 est équipé d'un filtre bactérien 16 et l'extrémité d'un cathéter 17 est fixée par collage dans celui-ci. Le corps 11 et sa membrane 14 peuvent être entièrement recouverts d'un revêtement souple en silicone (non représenté).

## Revendications

1. Dispositif destiné à être implanté dans une zone sous-cutanée accessible en vue de constituer un site de perfusion et/ou d'injection chronique d'une substance liquide, comprenant:
   un corps rigide monobloc (1, 11) d'épaisseur plus faible que ses deux autres dimensions, et de forme permettant de le glisser dans la zone sous-cutanée,
   ledit corps étant creusé dans son épaisseur d'une dépression (2, 12) concsve arrondie, apte à faire office de réservoir de substance liquide,
   le corps étant percé d'un canal (5, 15) débouchant dans le fond de la dépression (2, 12) dans la partie la plus profonde de celle-ci par un orifice de communication,
   ledit canal s'étendant dans l'épaisseur du corps de façon à déboucher à l'extérieur sur le pourtour dudit corps,
   un cathéter souple (7, 17) étant fixé de façon étanche par son extrémité dans ledit canal de façon à se prolonger à l'extérieur du corps,
   le corps étant pourvu autour de la dépression d'une bordure périphérique (3, 13),
   une membrane élastique (4, 14) de type auto-étanche sensiblement plane à l'état de repos étant fixée de façon étanche sur ladite bordure périphérique,
   la dépression (2, 12) s'ouvrant sur une face du corps (1a, 11a) et présentant une profondeur faible par rapport aux dimensions de sa section d'ouverture, de façon que la membrane vienne épouser le fond de la dépression lorsqu'elle est repoussée par l'index de l'utilisateur, ledit dispositif étant caractérisé en ce que la membrane est positionnée sur le corps en position inclinée par rapport à la face inférieure de celui-ci (1b, 11b), et en ce que ledit corps présente une épaisseur décroissante en direction de l'orifice de communication du canal dans la dépression.

2. Dispositif selon la revendication 1, dans lequel le corps et la membrane élastique sont entièrement recouverts d'un revêtement souple (8) en une matière biocompatible, en particulier en silicone.

3. Dispositif selon l'une des revendications 1 ou 2, dans lequel une valve antimicrobienne (6) est disposée dans le canal (5, 15) du corps, immédiatement en amont de l'extrémité du cathéter (7, 17).

4. Dispositif selon l'une des revendications 1, 2 ou 3, caractérisé en ce que le canal (5, 15) est percé dans l'épaisseur du corps de sorte que le cathéter sorte du corps selon une direction approximativement parallèle au plan de l'une de ses faces (1b, 11b).

5. Dispositif selon l'une des revendications 1, 2, 3 ou 4, caractérisé en ce que la dépression (2, 12) possède une forme circulaire ou oblongue, symétrique par rapport à un plan longitudinal (AA'), le fond de ladite dépression étant incliné dans le sens longitudinal vers l'orifice du canal (5, 15).

6. Dispositif selon l'une des revendications 1, 2, 3, 4 ou 5, caractérisé en ce que le corps (1, 11) est moulé d'un seul tenant en matière synthétique rigide biocompatible, notamment polycarbonate ou silicone renforcé.

7. Dispositif selon l'une des revendications 1, 2, 3, 4, 5 ou 6, caractérisé en ce que la membrane (4, 14) est découpée à la forme de la bordure périphérique (3, 13) de la dépression dans une matière synthétique élastique biocompatible du type ''auto-étanche'', notamment silicone.

8. Dispositif selon l'une des revendications précédentes, dans lequel la dépression (2, 12) possède un volume compris entre 0,2 et 1,7 cm$^3$ en vue de délimiter avec la membrane au repos une chambre de capacité correspondant à une dose de substance thérspeutique.

9. Dispositif selon l'une des revendications précédentes, pourvu de part et d'autre de l'extrémité du cathéter (7, 17), de deux trous de suture (9, 19) traversant son épaisseur.

## Patentansprüche

1. Vorrichtung zur Implantation in eine zugängliche subkutane Zone zwecks Erzielung eines Bereichs für wiederholte perfusion und/oder Zuführung einer flüssigen Substanz, umfassend:

einen starren aus einem Stück bestehenden Körper (1, 11) mit einer Dicke, die geringer ist als dessen beide andere Dimensionen und dessen Form die Möglichkeit bietet ihn in die subkutane Zone einzuschieben,

wobei der besagte Körper ausgehöhlt ist, so daß er in seiner Dicke einen gerundeten konkaven Hohlraum (2, 12) enthält, der als Behälter für flüssige Substanz dienen kann,

wobei ein Kanal (5, 15), der über eine Kommunikationsöffnung in dem Boden des Hohlraums (2, 12) mündet, und zwar in dessen tiefstem Bereich, durch den Körper hindurchführt,

wobei sich der besagte Kanal innerhalb der Dicke des Körpers so erstreckt, daß er am Umfang des besagten Körpers nach außen austritt,

wobei ein flexibler Katheter (7, 17) auf dichte Weise mit seinem Ende in dem besagten Kanal befestigt ist, so daß er sich außerhalb des Körpers erstreckt,

wobei der Körper rings um den Hohlraum mit einem peripheren Rand (3, 13) versehen ist,

wobei eine elastische Membran (4, 14) von selbstdichtender Art, die im Ruhezustand im wesentlichen flach ist, auf dichte Weise an dem peripheren Rand befestigt ist,

wobei sich der Hohlraum (2, 12) auf eine Fläche des Körpers (1a, 11a) zu erweitert und im Verhältnis zu den Abmessungen des Erweiterungsquerschnitts eine geringe Tiefe besitzt, so daß sich die Membran an dem Boden des Hohlraums anlegt, wenn sie durch den Zeigefinger des Benutzers niedergedrückt wird, wobei die besagte Vorrichtung dadurch gekennzeichnet ist, daß die Membran in im Verhältnis zu dessen unterer Fläche (1b, 11b) geneigter Lage an dem Körper angeordnet ist, sowie dadurch, daß der besagte Körper eine in der Richtung der Kommunikationsöffnung des Kanals innerhalb des Hohlraums abnehmende Dicke aufweist.

2. Vorrichtung nach Anspruch 1, bei der der Körper und die flexible Membran völlig mit einer flexiblen Außenschicht (8) aus einem biokompatiblen Material, insbesondere Silikon, überzogen ist.

3. Vorrichtung nach einem der Ansprüche 1 oder 2, bei der ein Antimikrobenventil (6) innerhalb des Kanals (5, 15) des Körpers angeordnet ist, und zwar unmittelbar vor dem Ende des Katheters (7, 17).

4. Vorrichtung nach einem der Ansprüche 1, 2 oder 3, dadurch gekennzeichnet, daß der Kanal (5, 15) so durch die Dicke des Körpers hindurchführt, daß der Katheter in einer Richtung aus dem Körper austritt, die zu der Ebene von einer der Flächen des Körpers (1b, 11b) annahernd parallel ist.

5. Vorrichtung nach einem der Ansprüche 1, 2, 3 oder 4, dadurch gekennzeichnet, daß der Hohlraum (2, 12) eine runde oder längliche Form besitzt, die im Verhältnis zu einer Längsebene (AA') symmetrisch ist, wobei der Boden des besagten Hohlraums in Längsrichtung auf die Öffnung des Kanals (5, 15) zu geneigt ist.

6. Vorrichtung nach einem der Ansprüche 1, 2, 3, 4 oder 5, dadurch gekennzeichnet, daß der Körper (1, 11) in einem Gang aus einem biokompatiblen starren Kunststoff, insbesondere verstärktem Polykarbonat oder Silikon, geformt wird.

7. Vorrichtung nach einem der Ansprüche 1, 2, 3, 4, 5 oder 6, dadurch gekennzeichnet, daß die Membran (4, 14) der Form des peripheren Randes (3, 13) des Hohlraums entsprechend aus einem biokompatiblen elastischen Kunststoff "selbstdichtender" Art, insbesondere Silikon, zugeschnitten ist.

8. Vorrichtung nach einem der vorstehenden Ansprüche, bei der der Hohlraum (2, 12) ein Volumen zwischen 0,2 und 1,7 cm$^3$ besitzt, um mit der im Ruhezustand befindlichen Membran eine Kammer zu bilden, deren Fassungsvermögen einer Dosis der therapeutischen Substanz entspricht.

9. Vorrichtung nach einem der vorstehenden Ansprüche mit je einem zu beiden Seiten des Katheterendes (7, 17) befindlichen Nahtloch (9, 19), das durch die Dicke der besagten Vorrichtung hindurchführt.

## Claims

1. Device for implantation in an accessible subcutaneous zone intended to constitute a site for regular perfusion and/or injection of a liquid substance with:

a rigid body consisting of a single piece (1, 11) the thickness of which is smaller than its two other dimensions and of a form enabling it to be slid into the subcutaneous zone,

said body being hollowed out in its thickness so as to form a rounded concave cavity (2, 12) capable of serving as a reservoir for liquid substance,

the body being penetrated by a duct (5, 15) leading into the base of the cavity (2, 12) at the lowest part of the latter via a communication orifice,

said duct extending within the thickness of the body in such a way as to emerge outside at the periphery of said body,

a flexible catheter (7, 17), the end of which is fixed in sealed manner within said duct, so as to extend outside the body,

the body being provided with a peripheral border (3, 13) about the cavity,

an elastic membrane (4, 14) of self-sealing type, which in the state of rest is substantially plane, being fixed in sealed manner to said peripheral border,

the cavity (2, 12) opening out towards one face of the body (1a, 11a) and presenting a depth which by comparison with the dimensions of the opening section is small, so that the membrane is adapted to the bottom of the cavity when it is depressed by the user's index, said device being characterised in that the membrane is arranged on the body in a position inclined in relation to the lower face of the latter (1b, 11b) and in that said body Presents a diminishing thickness in the direction of the communication orifice of the duct within the cavity.

2. Device according to claim 1 in which the body and the elastic membrane are entirely clad with a flexible lining (8) made from biocompatible material, in particular silicone.

3. Device according to one of claims 1 or 2, in which an antimicrobial valve (6) is arranged within duct (5, 15) of the body, directly upstream of the end of the catheter (7, 17).

4. Device according to one of claims 1, 2 or 3, characterised in that the duct (5, 15) penetrates the thickness of the body so that the catheter emerges from the body in a direction approximately parallel to the plane of one of its faces (1b, 11b).

5. Device according to one of claims 1, 2, 3 or 4, characterised in that the cavity (2, 12) has a circular or oblong shape symmetrical in relation to a longitudinal plane (AA'), the bottom of said cavity being inclined in the longitudinal direction towards the orifice of the duct (5, 15).

6. Device according to one of claims 1, 2, 3, 4 or 5, characterised in that the body (1, 11) is moulded in a single operation from rigid biocompatible synthetic material, in particular reinforced polycarbonate or silicone.

7. Device according to one of claims 1, 2, 3, 4, 5 or 6, characterised in that the membrane (4, 14) is cut to the shape of the peripheral border (3, 13) of the cavity, from a synthetic, elastic and biocompatible material of "self-sealing" type, in particular silicone.

8. Device according to one of the preceding claims, in which the cavity (2, 12) has a volume between 0.2 and 1.7 $cm^3$, so as to delimit together with the membrane in its state of rest a chamber with a capacity corresponding to one dose of therapeutic substance.

9. Device according to one of the preceding claims, provided on one side and on the other of the end of the catheter (7, 17) with two suture holes (9, 19) passing through its thickness.

Fig. 1

Fig. 2

Fig. 3

Fig. 4

Fig. 5

Fig. 6

Fig. 7a

Fig. 7b

R

Fig. 7c

Fig. 8

Fig. 9